# EUROPEAN PATENT SPECIFICATION

(11) **EP 2 153 861 B1**
(45) Date of publication and mention of the grant of the patent: **06.03.2013**
(21) Application number: 07740638.7
(22) Date of filing: 30.03.2007
(51) Int. Cl.: A61M 25/09

(54) **MEDICAL GUIDE WIRE**
MEDIZINISCHER FÜHRUNGSDRAHT
FIL DE GUIDAGE MÉDICAL

(43) Date of publication of application: 17.02.2010
(73) Proprietor: Piolax Medical Devices, Inc., Yokohama-shi Kanagawa 240-0023 (JP)
(72) Inventor: TAKAHASHI, Hideo, Yokohama-shi Kanagawa 240-0023 (JP); ISAYAMA, Hiromichi, Tokyo 152-0001 (JP)
(74) Representative: Grünecker, Kinkeldey, Stockmair & Schwanhäusser
(86) International application number: PCT/JP2007/057202
(87) International publication number: WO 2008/126286

(56) References cited:
- EP-A1- 1 203 595
- JP-A- 2001 046 508
- JP-A- 2001 046 508
- JP-A- 2001 340 288
- JP-A- 2004 229 947
- US-A- 5 379 779

## Description

### Technical Field

The present invention relates to a medical guide wire used for guiding a leading end of a catheter to a target place when the catheter is inserted into a human body's tubular organ such as a blood vessel, a ureter, a bile duct, a trachea, etc.

### Background Art

In recent years, in a state in which a catheter is inserted, a medicine such as a contrast medium is administered or a forceps etc. is operated to take a part of a tissue through the catheter for inspection/treatment in a human body's tubular organ such as a blood vessel, a ureter, a bile duct, a trachea, etc. For insertion of the catheter, a comparatively thin and flexible guide wire is first inserted into the tubular organ so that a leading end of the guide wire reaches a target place, and then the catheter is inserted along an outer circumference of the guide wire and the guide wire is pulled out.

For example, in treatment using a balloon catheter, the guide wire needs to be held so as not to move when the balloon catheter is exchanged. While an image of the inside is picked up through an endoscopic fiberscope, the work is made so that the position of the leading end portion of the guide wire is not displaced during exchange of the balloon catheter.

For this reason, when visibility of the guide wire is poor, displacement, etc. of the position of the leading end portion of the guide wire sometimes occurs during exchange of the balloon catheter so that the balloon catheter cannot be indwelled in the target place accurately.

Therefore, various attempts have been made so that visibility of the guide wire due to the fiberscope is improved when the guide wire is inserted into the human body or pulled out of the human body. For example, Patent Document 1 listed below has disclosed a medical guide wire provided with a core material having a leading end portion with a smaller diameter than that of a base side, an intermediate portion continuing to the leading end portion and extending to the base side, and a base end portion continuing to the intermediate portion and further extending to the base side, wherein: a synthetic resin film is applied on the core material; a helical pattern is formed in the synthetic resin film at least applied on the intermediate portion; and at least one of the synthetic resin film applied on the leading end portion and the synthetic resin film applied on the base end portion has a portion in which the pattern is not formed.

Patent Document 1: JP-A-2001-046508

### Disclosure of the Invention

### Problem that the Invention is to Solve

Even in the guide wire according to the Patent Document 1, visibility due to the fiberscope is provided but its effect is insufficient yet.

Moreover, although the outer circumferential portion of the guide wire is coated with a hydrophilic resin to reduce frictional resistance between the guide wire and a tube such as a catheter, a cannula, etc. and improve slidability, it cannot be said that slidability of the guide wire is sufficient. Accurate operation is sometimes difficult because workability in inserting these tubes is inferior.

Therefore, an object of the invention is to provide a guide wire which is excellent in visibility due to a fiberscope in a human body and which is low in frictional resistance to tubes such as a catheter, a cannula, etc. so as to be excellent in inserting workability.

### Means for Solving the Problem

To achieve the foregoing object, the invention provides a guide wire in which at least one part of an outer circumference of a core wire is coated with a resin film having uneven helical patterns distinguished by colors.

According to the invention, because at least one part of an outer circumference of a core wire is coated with a resin film having uneven helical patterns distinguished by colors, the helical patterns distinguished by colors are given to at least one part of the outer circumference of the guide wire by the resin film. For this reason, when work due to a fiberscope is performed, the helical patterns distinguished by colors can be used as markers to confirm the direction of movement of the guide wire, the distance of the movement, etc., so that visibility for the motion of the guide wire can be improved.

Moreover, because the outer circumference of the guide wire is made uneven by the helical patterns, convex portions of the helical patterns of the outer circumference of the guide wire come into contact with a tube such as a catheter, a cannula, etc. when the guide wire attached to the tube is used. Accordingly, because the area of contact between the guide wire and the tube is reduced so that frictional resistance in insertion of the tube can be reduced, workability in inserting the tube can be improved.

In the invention, it is preferable that the unevenness has a depth of from 0.01mm to 0.1mm. By setting the depth of the unevenness to be in the aforementioned range, the resin film can be kept relatively thin to reduce the area of contact between the guide wire and a tube such as a catheter, a cannula, etc. without spoiling the flexibility and thinness of the guide wire to reduce frictional resistance between the guide wire and the tube to thereby improve the slidability of the guide wire.

In the invention, it is also preferable that the helical patterns are provided at intervals of a pitch of from 1/cm to 5/cm. According to this configuration, because the helical patterns are provided at intervals of a relatively short pitch, frictional resistance between the guide wire and the tube can be reduced to improve the slidability of the guide wire and the number of helical patterns existing in an image region observed by a fiberscope can be increased so that the traveling state, the distance of movement, etc. of the guide wire can be confirmed more accurately and in detail.

In the invention, it is further preferable that the resin film having the uneven helical patterns is applied at least on a range of from a position 30mm distant from a foremost leading end portion of the guide wire to a position 500mm distant from the foremost leading end portion. Because the range of from a position 30mm distant from the foremost leading end portion of the guide wire to a position 500mm distant from the foremost leading end portion is a portion most frequently viewed by the fiberscope when the guide wire is operated, at least coating this portion with the resin film having the uneven helical patterns is more suitable for confirming the traveling state, the distance of movement, etc. of the guide wire.

### Advantages of the Invention

According to the guide wire of the invention, because the helical patterns distinguished by colors are given to at least one part of the outer circumference of the guide wire by the resin film having the uneven helical patterns distinguished by colors, the helical patterns distinguished by colors can be used as markers to confirm the direction of movement of the guide wire, the distance of the movement, etc. to thereby improve visibility for the motion of the guide wire when work using a fiberscope is performed. Moreover, because at least one part of the outer circumference of the guide wire is made uneven by the helical patterns, the area of contact between the guide wire and a tube such as a catheter, a cannula, etc. can be reduced when the guide wire inserted onto the tube is used. Accordingly, frictional resistance in insertion/removal of the tube can be reduced to improve workability in insertion of the tube.

### Best Mode for Carrying out the Invention

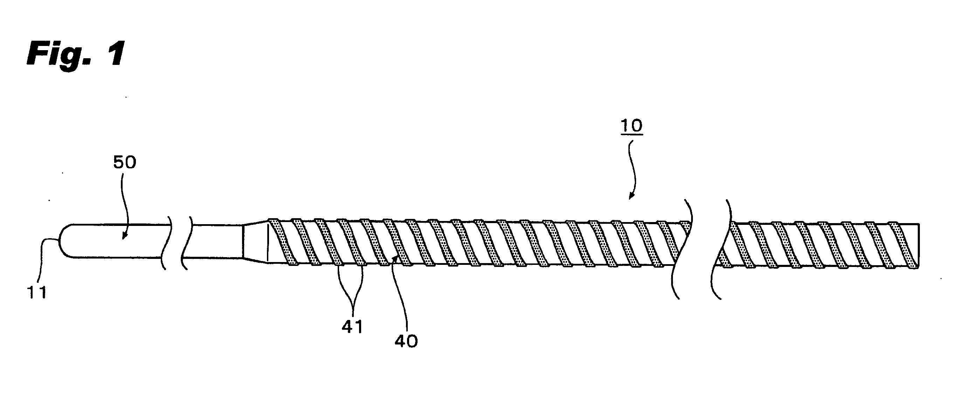

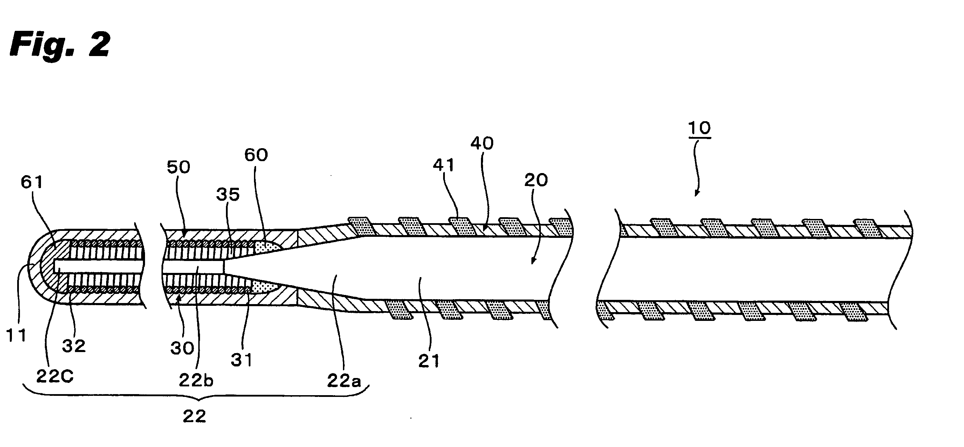

An embodiment of a guide wire according to the invention will be described below with reference to Figs. 1 and 2.

As shown in Figs. 1 and 2, the guide wire 10 mainly includes a core wire 20, a coil 30 wound on an outer circumference of a leading end portion 22 of the core wire 20, a first resin film 40 with which a base portion 21 side of the core wire 20 is coated, and a second resin film 50 applied on an outer circumference of the coil 30 so that the leading end portion 22 is coated with the second resin film 50.

As shown in Fig. 2, the core wire 20 has a base portion 21, and a leading end portion 22 tapered from the base portion 21. The leading end portion 22 further has a taper portion 22a tapered from the base portion 21, a reduced diameter portion 22b extending from the taper portion 22a, and a foremost leading end 22c.

The leading end portion 22 can be formed by means of machining, etching, etc. The shape of the leading end portion 22 is not limited to the shape shown in Fig. 2. For example, a shape of steps formed by reducing the diameter stepwise from the base portion 21 may be used but the shape is not particularly limited.

A radiopaque material selected from the group consisting of superelastic alloy such as Ni-Ti alloy, Ni-Ti-X (X=Fe, Cu, V, Co, etc.) alloy or Cu-Zn-X (X=Al, Fe, etc.), stainless steel, piano wire material, metal such as Pt, Ti, Pd, Rh, Au or W, and alloy thereof is preferably used as the material of the core wire 20.

The coil 30 is disposed on the outer circumference of the leading end portion 22 of the core wire 20. In this embodiment, a base end portion side 31 of the coil 30 is joined to an intermediate portion of the taper portion 22a by a brazing material 60, and a leading end portion side 32 of the coil 30 is joined to the foremost leading end 22c of the core wire 20 by a brazing material 61.

The same material as that of the core wire 20 can be used as the material of a wire material forming the coil 30.

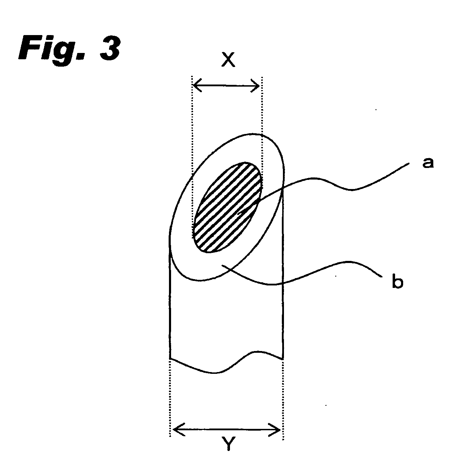

Incidentally, a wire material formed from a superelastic alloy b shaped like a tube and disposed on an outer circumference, and a radiopaque material a disposed in a central portion thereof as shown in Fig. 3 is particularly preferred as the wire material of the core wire 20 and/or the coil 30. In this case, the superelastic alloy b disposed on the outer circumference and the radiopaque material a disposed in the central portion may be integrated with each other or may be provided so separately as to be relatively movable in an axial direction.

A metal such as Au, Pt, Ag, Bi or W, an alloy containing these metals, or the like, is used as the radiopaque material a disposed in the central portion. An Ni-Ti type shape memory alloy or the like is preferably used as the superelastic alloy b disposed on the outer circumference. The relation between the diameter X of the radiopaque material a disposed in the central portion and the diameter Y of the wire material in Fig. 6 is preferably set so that the cross sectional area of the radiopaque material a disposed in the central portion is in a range of from 10% to 40% of the cross sectional area of the wire material.

Because the wire material is formed from the radiopaque material a disposed in the central portion and the superelastic alloy b disposed on the outer circumference, the wire material has both flexibility that the wire material can be bent naturally in accordance with a bent portion of the tubular organ and visibility that the position of the coil 30 can be viewed by radiocontrast using an X-ray fluoroscopic camera or the like.

The base portion 21 side of the core wire 20 is coated with the first resin film 40. In this embodiment, the first resin film 40 partially covers up to an intermediate portion of the taper portion 22a of the core wire 20.

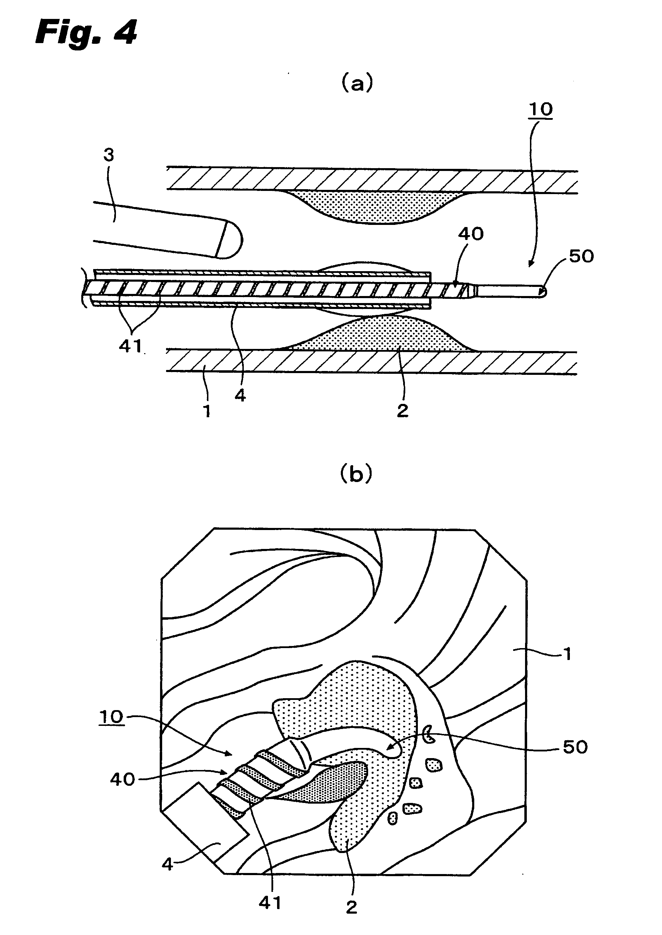

The first resin film 40 further has helical patterns 41 formed along an axial direction thereof. The helical patterns 41 separate the first resin film 40 into colors to thereby improve visibility when the guide wire 10 is operated while an image of the guide wire 10 is displayed on a monitor by a fiberscope 3 (see Fig. 4) which will be described later. At the same time, the helical patterns 41 form unevenness on a surface of the first resin film 40 to reduce the area of contact with a tube such as a catheter, a cannula, etc. to thereby improve the slidability of the guide wire.

For example, the first resin film 40 can be formed by a method of molding a resin tube having uneven helical patterns by extruding two-color heat-shrinkable resin materials simultaneously from rotating cylindrical mouthpieces while changing extrusion pressures of the resin materials respectively and heat-shrinking the resin tube applied on the outer circumference of the base portion 21 side of the core wire 20, or by a method of directly coating the outer circumference of the base end 21 side of the core wire 20 with the resin film 40 by the aforementioned extrusion molding method.

Examples of the material of the first resin film 40 include: fluorocarbon resins such as polytetrafluoroethylene (PTFE), perfluoroalkoxy resin (PFA), ethylene tetrafluoride-propylene hexafluoride copolymer (FEP), ethylene tetrafluoride-ethylene copolymer (ETFE), etc.; and synthetic resins such as polyurethane, Nylon elastomer, polyether block amide, polyethylene, poly(vinyl chloride), vinyl acetate, etc. Fluorocarbon resins such as polytetrafluoroethylene (PTFE), etc. are preferred. Powder of BaSO4, Bi, W or the like may be contained in the aforementioned resin material to provide the resin material as a radiopaque material.

The depth of the unevenness formed from the helical patterns 41 is preferably in a range of from 0.01mm to 0.1mm, more preferably in a range of from 0.02mm to 0.03mm. If the depth of the unevenness is smaller than 0.01mm, the effect of reducing frictional force between the guide wire and the tube such as a catheter, a cannula, etc. runs low and the slidability of the guide wire is insufficient. If the depth of the unevenness is larger than 0.1mm, the outer diameter of the guide wire becomes so large that workability in inserting the guide wire into the human body is lowered.

The helical patterns 41 are provided preferably at intervals of a pitch of from 1/cm to 5/cm, especially preferably at intervals of a pitch of 2/cm to 3/cm. On this occasion, the term "a pitch" means the length of one repeating unit of the uneven helical patterns. For example, it means the distance between the center of one convex portion and the center of a next adjacent convex portion. The term "from 1/cm to 5/cm" means the interval in which the number of the repeating units per 1 cm is from 1 to 5. When the guide wire has the helical patterns provided at intervals of the aforementioned pitch, the guide wire can be excellent in visibility due to a fiberscope or the like and low in frictional resistance to a tube such as a catheter, a cannula, etc. to provide good slidability.

The length of a range to which the helical patterns 41 are attached is preferably in a range of from 450mm to 5000mm, more preferably in a range of from 450mm to 4500mm. If the length is smaller than 450mm, a range in which confirmation of position, measurement of distance, etc. can be performed by a fiberscope is shortened. If the length is larger than 5000mm, production cost becomes high wastefully because the helical patterns must be attached even to a portion not used for confirmation by the fiberscope.

The helical patterns 41 are preferably formed at least in a range of from a position 30mm distant from the foremost leading end portion 11 of the guide wire 10 to a position 500mm distant from the foremost leading end portion 11, more preferably formed at least in a range of from a position 50mm distant from the foremost leading end portion 11 of the guide wire 10 to a position 500mm distant from the foremost leading end portion 11. As a range used for confirmation due to a fiberscope, the range of from a position 30mm distant from the foremost leading end portion 11 of the guide wire 10 to a position 500mm distant from the foremost leading end portion 11 is a portion most frequently used.

The leading end portion 22 side of the core wire 20 is coated with the second resin film 50.

The second resin film 50 is formed of, for example, a fluorocarbon resin such as polytetrafluoroethylene (PTFE), perfluoroalkoxy resin (PFA), ethylene tetrafluoride-propylene hexafluoride copolymer (FEP), ethylene tetrafluoride-ethylene copolymer (ETFE), etc., or a synthetic resin such as polyurethane, Nylon elastomer, polyether block amide, polyethylene, poly(vinyl chloride), vinyl acetate, etc. The second resin film 50 is preferably formed of a synthetic resin such as polyurethane etc. excellent in flexibility. Powder of BaSO4, Bi, W or the like may be contained in the aforementioned resin material to provide the resin material as a radiopaque material.

The outer circumference of each of the first and second resin films 40 and 50 is preferably coated with a hydrophilic resin such as polyvinylpyrrolidone, polyethylene glycol, methyl vinyl ether-maleic anhydride copolymer, etc. to give slidability and thrombus anti-adhesiveness.

An example of a method of producing the guide wire 10 according to the invention will be described below.

First, the coil 30 is disposed on the outer circumference of the leading end portion 22 of the core wire 20. While the base end portion side 31 of the coil 30 is fixed to an intermediate portion of the taper portion 22a of the core wire 20 by a brazing material, the leading end portion side 32 of the coil 30 is fixed and joined to the foremost leading end 22c of the core wire 20 by a brazing material 61.

A first tube serving as the first resin film 40 formed with an inner diameter slightly larger than the outer diameter of the base portion 21 of the core wire 20 and having an end surface cut by a cutter or the like is inserted from the base portion 21 side of the core wire 20 so that the leading end portion of the first tube forcedly reaches the base end of the leading end 22 of the core wire 20.

The first tube is heated and heat-shrunk by heating means such as a heater so that the base portion 21 of the core wire 20 is coated with the first film 40.

Then, an acrylic adhesive agent such as a cyanoacrylate adhesive or an epoxy adhesive agent is applied on an inner circumference of a leading end portion and an end surface of the first resin film 40 and a second tube serving as the second resin film is inserted onto the outer circumferences of the leading end portion 22 of the core wire 20 and the coil 30. Incidentally, the second tube is swollen by a solvent such as methyl ethyl ketone (MEK) in advance.

In the state where the second tube is inserted, the solvent is dried to shrink the second tube so that the base end of the second tube is fixed to the leading end of the first resin film 40 by the adhesive agent while the inner circumference of the second tube is fixed to the outer circumferences of the core wire 20 and the coil 30, so that the leading end 22 side of the core wire 20 is coated with the second resin film 50.

An example of usage of the guide wire 10 according to the invention will be described below with reference to Figs. 4 (a) and 4(b). Fig. 4 (a) is an explanatory view showing a use state of the guide wire. Fig. 4(b) is an explanatory view showing a use state due to a fiberscope.

For example, the guide wire 10 is inserted into a bile duct 1 or the like via a duodenum and used for treating a diseased place such as a stenosed portion 2.

First, a fiberscope 3, the guide wire 10 according to the invention and a balloon catheter 4 along the outer circumference of the guide wire 10 are inserted through lumina formed in a tubular carrier not shown. While the helical patterns 41 of the first resin tube 40 are used so that an image from the fiberscope 3 is confirmed by an endoscope, the carrier is inserted into the duodenum via the oral cavity and further inserted into the bile duct 1.

When the guide wire 10 reaches the stenosed portion 2 as a target place, the balloon catheter 4 is inserted along the outer circumference of the guide wire 10. When the inserted balloon catheter 4 reaches the stenosed portion 2, the guide wire 10 is pulled out and the balloon of the balloon catheter is swollen with physiological saline or the like so that the stenosed portion 2 is pressed from the inside so as to be widened. On this occasion, because the area of contact between the guide wire and the balloon catheter is made small by the presence of the uneven helical patterns 41, the slidability of the guide wire 10 and the balloon catheter 4 is so high that workability in insertion of the balloon catheter 4 is good.

### Embodiments

The invention will be described below further in detail in conjunction with embodiments.

### (Embodiment 1)

A guide wire according to Embodiment 1 was produced in such a manner that a resin tube made of polytetrafluoroethylene (PTFE) and having 0.01mm-deep uneven helical patterns 41 distinguished by two colors and disposed at intervals of a pitch of 1/cm was applied on an outer circumference of a core wire 20, and the outer circumference of the core wire 20 was coated with the resin tube by heat-shrinking due to a heater.

### (Embodiment 2)

A guide wire according to Embodiment 2 was produced in the same manner as in Embodiment 1, except that a resin tube made of polytetrafluoroethylene (PTFE) and having 0.02mm-deep uneven helical patterns 41 distinguished by two colors and disposed at intervals of a pitch of 5/cm was used.

### (Test Example)

The cannula insertion maximum load (N) of each of the guide wires according to Embodiments 1 and 2 was measured in such a manner that a portion 10cm distant from the leading end of the guide wire was fixed and attached to a load measuring device (tradename; "DIGITAL FORCE GAUGE", model number; "AD-4935-200N", manufacturer; "A&D") and inserted onto a cannula (tradename "XEMEX ERCP catheter" made by ZEON CORPORATION) disposed in the leading end portion, by a stroke length of 10cm at a pulling-out velocity of 500mm/min. Results thereof are shown in Table 1.

**[Table 1]**

| | Cannula Insertion Load (N) |
|---|---|
| Embodiment 1 | 0.73 |
| Embodiment 2 | 0.63 |

As shown in Table 1, as the pitch of the uneven helical patterns was narrowed, the cannula insertion load was reduced so that frictional resistance could be reduced.

### Brief Description of the Drawings

Fig. 1 is an explanatory view showing an embodiment of a guide wire according to the invention.
Fig. 2 is a sectional view of a leading end portion of the guide wire.
Fig. 3 is an explanatory view showing another example of wire material forming a core wire and a coil of the guide wire.
Fig. 4 (a) is an explanatory view showing a use state of the guide wire, and Fig. 4(b) is an explanatory view showing a use state of the guide wire due to a fiberscope.

### Description of Numerals

- 10:: guide wire
- 20:: core wire
- 30:: coil
- 40:: first resin film
- 41:: helical pattern
- 50:: second resin film
- 60, 61:: brazing material

## Claims

1. A medical guide wire comprising a core wire (20) configured by a base portion (21) having substantially equal diameter and a leading end portion (22) being At tapered from the base portion (21). At least one part of an outer circumference of the core wire (20) is coated with a resin film (40), **characterized in that** the coated film is defined by an uneven helical pattern (41) being colored in a plurality of colors.

2. The medical guide wire according to Claim 1, wherein a depth of the helical pattern (41) is set in a range from 0.01 mm to 0.1mm.

3. The medical guide wire according to Claim 1, wherein the helical pattern (41) is provided at intervals of a pitch in a range from 1/cm to 5/cm.

4. The medical guide wire according to Claim 2, wherein the helical pattern (41) is provided at intervals of a pitch in a range from 1/cm to 5/cm.

5. The medical guide wire according to Claim 1, wherein the resin film (40) having the uneven helical pattern (41) is applied at least on a range of from a position 30mm distant from a foremost leading end portion (22c) of the guide wire (10) to a position 500mm distant from the foremost leading end portion (22c).

## Patentansprüche

1. Medizinischer Führungsdraht, der einen Kerndraht (20), der durch einen Basisabschnitt (21), der einen im Wesentlichen einheitlichen Durchmesser hat, und einen vorderen Endabschnitt (22) gebildet wird, der sich von dem Basisabschnitt (21) aus verjüngt. Wenigstens ein Teil eines Außenumfangs des Kerndrahtes (20) ist mit einem Kunststofffilm (40) beschichtet, **dadurch gekennzeichnet, dass** der Beschichtungsfilm durch ein ungleichmäßiges schraubenförmiges Muster (41) gebildet wird, das in einer Vielzahl von Farben gefärbt ist.

2. Medizinischer Führungsdraht nach Anspruch 1, wobei eine Tiefe des schraubenförmigen Musters (41) auf einen Bereich von 0,01 mm bis 0,1 mm festgelegt ist.

3. Medizinischer Führungsdraht nach Anspruch 1, wobei das schraubenförmige Muster (41) in Intervallen einer Steigung in einem Bereich von 1/cm bis 5/cm vorhanden ist.

4. Medizinischer Führungsdraht nach Anspruch 2, wobei das schraubenförmige Muster (41) in Intervallen einer Steigung in einem Bereich von 1/cm bis 5/cm vorhanden ist.

5. Medizinischer Führungsdraht nach Anspruch 1, wobei der Kunststofffilm (40), der das ungleichmäßige schraubenförmige Muster (41) hat, in wenigstens einem Bereich von einer Position, die 30 mm von einem vordersten Endabschnitt (22c) des Führungsdrahtes (10) entfernt ist, bis zu einer Position aufgebracht ist, die 500 mm von dem vordersten Endabschnitt (22c) entfernt ist.

## Revendications

1. Fil guide médical comprenant une tige d'armature (20) configurée par une partie de base (21) ayant un diamètre sensiblement égal et une partie d'extrémité d'attaque (22) qui est progressivement rétrécie à partir de la partie de base (21), au moins une partie d'une circonférence externe de la tige d'armature (20) est recouverte avec un film de résine (40), **caractérisé en ce que** le film appliqué est défini par un modèle hélicoïdal irrégulier (41) qui est coloré avec une pluralité de couleurs.

2. Fil guide médical selon la revendication 1, dans lequel une profondeur du modèle hélicoïdal (41) est dans une plage de 0,01 mm à 0,1 mm.

3. Fil guide médical selon la revendication 1, dans lequel le modèle hélicoïdal (41) est prévu à intervalles avec un pas dans une plage de 1/cm à 5/cm.

4. Fil guide médical selon la revendication 2, dans lequel le modèle hélicoïdal (41) est prévu à intervalles avec un pas dans une plage de 1/cm à 5/cm.

5. Fil guide médical selon la revendication 1, dans lequel le film de résine (40) ayant un modèle hélicoïdal irrégulier (41) est appliqué au moins sur une plage allant d'une position à 30 mm à distance d'une partie d'extrémité d'attaque avant (22c) du fil guide (10) jusqu'à une position à 500 mm à distance de la partie d'extrémité d'attaque avant (22c).
